(19) Europäisches Patentamt
European Patent Office
Office européen des brevets

(11) Veröffentlichungsnummer: **0 022 498**
**A1**

# (12) EUROPÄISCHE PATENTANMELDUNG

(21) Anmeldenummer: **80103638.5**

(22) Anmeldetag: **27.06.80**

(51) Int. Cl.³: **C 07 D 307/86**, A 01 N 47/22, A 01 N 47/34, A 01 N 47/38

(30) Priorität: **13.07.79 DE 2928405**

(43) Veröffentlichungstag der Anmeldung: **21.01.81 Patentblatt 81/3**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Heywang, Gerhard, Dr., Nittumerweg 4, D-5060 Bergisch-Gladbach 2 (DE)**
Erfinder: **Hartmann, Alfons, Dr., Poststrasse 35, D-6645 Beckingen 4 (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**

(54) **Neue N-carboxylierte Carbamate, Verfahren zu ihrer Herstellung und ihre Verwendung.**

(57) Die vorliegende Erfindung betrifft neue N-carboxylierte N-Methyl-carbamate der Formel I

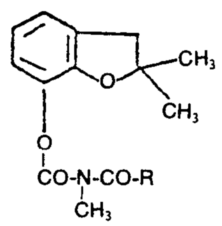

(I)

in welcher R durch ein Sauerstoff- oder Stickstoffatom gebundener organischer Rest bedeutet, ihre Herstellung durch Umsetzung des entsprechenden N-Chlorcarbonyl-benzofuranylcarbamats mit Alkoholen oder Aminen und ihre Verwendung als Insektizide.

EP 0 022 498 A1

BAYER AKTIENGESELLSCHAFT
Zentralbereich
Patente, Marken und Lizenzen

5090 Leverkusen, Bayerwerk
Rt/Kü/c
Typ Ia

Neue N-carboxylierte Carbamate, Verfahren zu ihrer Herstellung und ihre Verwendung

Die vorliegende Erfindung betrifft neue N-carboxylierte N-Methyl-carbamate, Verfahren zu ihrer Herstellung und ihre Verwendung als Schädlingsbekämpfungsmittel.

Es ist bereits bekannt geworden, daß N-carboxylierte N-Me= thylcarbamidsäurearylester (vergl. DE-OS 21 32 936) und N-Chlorcarbonyl-N-Methylcarbamidsäurearylester (vergl. DE-OS 21 42 496) insektizide Eigenschaften haben. Ihre Wirkung läßt jedoch, vor allem bei niedrigen Aufwandkon= zentrationen, vielfach zu wünschen übrig.

Es wurden nun neue N-carboxylierte N-Methylcarbamidsäure= arylester der Formel I gefunden.

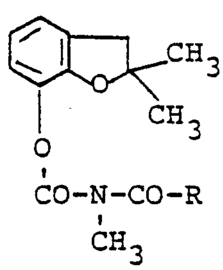

(I)

in welcher

R für einen geradkettigen oder verzweigten Alkoxyrest mit 1 - 10 C-Atomen, einen Alkenoxy- oder Alkinoxyrest mit 3 - 6 C-Atomen oder einen Cycloalkoxyrest mit 5 - 7 C-Atomen, die gegebenenfalls durch Halogen oder einen Rest der allgemeinen Formel $-X - R^1$ oder $-Y\ R^2\ R^3$ substituiert sein können; ferner steht R für $-Y\ R^2R^3$. In der Formel $-X - R^1$ steht X für das Sauerstoff- oder Schwefelatom, sowie für die Sulfoxid- oder Sulfongruppe und $R^1$ für Wasserstoff und/oder einen geradkettigen oder verzweigten Alkylrest mit 1 - 4 C-Atomen.

In der Formel $-Y\ R^2\ R^3$ steht Y für Stickstoff. $R^2$ und $R^3$ stehen für Wasserstoff und/oder verzweigte oder geradkettige Alkylreste, mit 1 -18 C-Atomen. Alkenylreste mit 3-8 C-Atomen, Alkinylreste mit 3-8 C-Atomen. Außerdem kann die Formel $-Y\ R^2R^3$ für einen cyclischen Aminorest stehen, der durch die allgemeinen Formeln

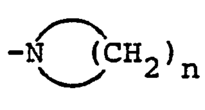 oder 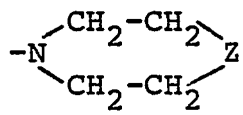 charakterisiert ist.

Hierbei steht n für 2 - 6 und Z für das Sauerstoff- oder Schwefelatom, sowie für ein wasserstoff- oder alkylsubstituiertes Stickstoffatom, wobei der Alkylsubstituent am Stickstoffatom 1 - 4 C-Atome haben kann.

Außerdem wurde gefunden, daß man die Verbindungen der Formel I erhält, wenn man N-Chlorcarbonyl-N-methyl-2,2-dimethyl-2,3-dihydrobenzofuranyl-(7)-carbamat der Formel

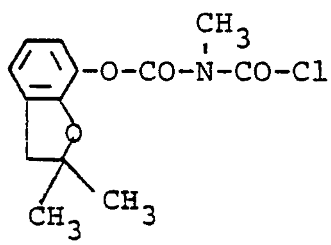 (II)

mit Alkoholen oder Aminen der Formel III,

R - H (III)

worin R die oben angegebenen Bedeutung hat,

in Gegenwart eines säurebindenden Mittels sowie gegebenenfalls eines Verdünnungsmittels umsetzt.

Es ist ausgesprochen überraschend, daß die erfindungsgemäßen Verbindungen eine höhere insektizide Potenz aufweisen als die eingangs erwähnten N-carboxylierten Carbamate.

Bevorzugt sind dabei Verbindungen in denen

R für $C_{1-4}$-Alkoxy, das gegebenenfalls durch $C_{1-4}$-Alkoxy, $C_{1-4}$-Alkylthio, Dialkylamino mit jeweils 1-4 C-Atomen pro Alkylrest, $C_{1-4}$-Alkylsulfinyl, $C_{1-4}$-Alkylsulfonyl, Halogen, insbesondere Cl oder F, Morpholino, Piperidino, oder OH, substituiert sein kann, sowie für Mono-oder Dialkylamino mit 1-18 C-Atomen pro Alkylrest, für Mono- oder Dialkenylamino mit 3-4 C-Atomen je Alkylrest, für Mono- oder Dialkinylamino mit 3-4 C-Atomen je Alkinylrest, für Morpholino, für Piperidino steht. Ferner steht R bevorzugt für $C_{3-4}$-Alkinoxy, $C_{3-4}$-Alkenoxy, sowie für $NH_2$.

In der allgemeinen Formel III steht R weiterhin bevorzugt für Aminoreste der allgemeinen Formel

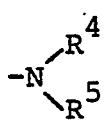 , 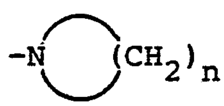 und 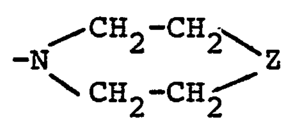

in welcher

$R^4$ und $R^5$ bevorzugt für Wasserstoff, geradkettige oder verzweigte Alkylreste mit 1-18 C-Atomen und geradkettige oder verzweigte Alkenylreste mit 3-8 C-Atomen stehen. (Die Reste $R^4$ und $R^5$ können in den Verbindungen der Formel III gleich oder verschieden sein).

n für die Zahlen 2 - 6 steht.

Z für das Sauerstoff- und das Schwefelatom, sowie für ein Sauerstoff-, Wasserstoff- oder alkylsubstituiertes Stickstoffatom steht; der Alkylsubstituent am Stickstoffatom kann 1 - 4 C-Atome haben.

Insbesondere seien die Verbindungen genannt in denen R für die Reste steht die weiter unten bei den Verbindungen der Formel III als bevorzugte Reste genannt sind.

Der Reaktionsablauf kann durch das folgende Formelschema wiedergegeben werden:

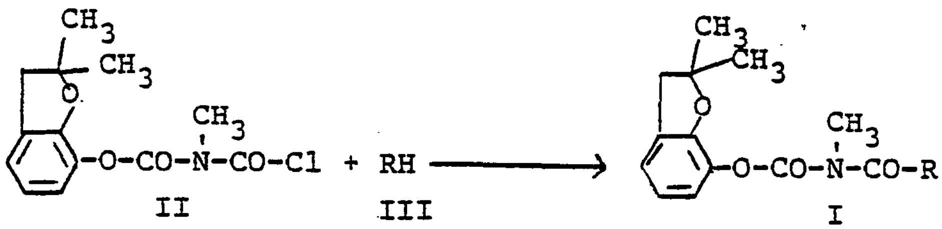

II III I

Die Ausgangskomponente II kann nach dem in der DE-OS 2 142 496 beschriebenen Verfahren hergestellt und entweder in reiner Form (Fp. 63°C) gewonnen oder im anfallenden Reaktionsgemisch ohne Isolierung weiter umgesetzt werden.

Die neuen Verbindungen, worin R für die Ethylsulfinylethoxy- und die Ethylsulfonylethoxygruppe steht, können auch durch Umsetzung der Verbindung II mit Ethylthioethanol und anschließender Oxidation mit Wasserstoffsuperoxid oder Persäuren gewonnen werden.

Bevorzugte Verbindungen der Formel III sind solche in denen R für Methoxy-, Ethoxy-, Propargyloxy-, Allyloxy-, Cyclohexyloxy-, Ethylthioethyloxy-, Chlorethoxy-, Trichlorethoxy-, Trifluorethoxy-, Dimethylaminoethoxy-, Piperidinoethoxy-, Morpholinopropoxy-, Propylsulfinylethoxy-, Ethylsulfonylethoxy- oder Ethoxyethoxy sowie deren Isomere steht.

Bevorzugte Verbindungen der Formel III sind ferner solche in denen R für Amino-, Methylamino-, Dimethylamino-, Ethylamino-, Diethylamino-, Dipropylamino-, Butylamino-, Dibutylamino-, Stearylamino-, Allylamino- Diallylamino-, Aziridino-, Pyrrolidino-, Morpholino-, Piperidino-, Piperazino Thiomorpholino-gruppen und deren Isomere steht.

Bevorzugt sind im besonderen die Verbindungen der Formel III worin R für die Methoxy-, Ethoxy-, Propargyloxy-, 2-Morpholino-1-methyl-ethoxy-, Piperidinoethoxy-, Pyrrolidino-ethoxy-gruppe, insbesondere vor allem für die 2-Chlorethoxy-, die 2,2,2-Trichlorethoxy-, 2,2,2-Trifluorethoxy-, 2-Dimethylamino-ethoxy-, 2-Ethoxy-ethoxy-, 2-Ethyl-thio-ethoxy-, 2-Ethylsulfinylethoxy- und die 2-Ethylsulfonylethoxygruppe steht.

Als Verdünnungsmittel für die erfindungsgemäßen Darstellungen eignen sich alle inerten organischen Lösungsmittel. Hierzu gehören Ether wie Diethylether, Dioxan oder Tetrahydrofuran, Kohlenwasserstoffe wie Benzol oder Toluol, Chlorkohlenwasserstoffe wie Dichlormethan, Chloroform oder Chlorbenzol, weiterhin Nitrile, Ester, Ketone sowie Gemische aus diesen Lösungsmitteln.

Als säurebindendes Mittel setzt man dem Reaktionsgemisch Natriumcarbonat, vorzugsweise eine tertiäre organische Base wie z.B. Triethylamin oder Benzyldimethylamin zu.

Die Reaktionstemperatur kann in einem größeren Bereich variiert werden. Im allgemeinen arbeitet man zwischen 0 und 100°C, vorzugsweise bei 10 - 60°C.
Gewöhnlich werden die Reaktionspartner in äquimolaren Mengen eingesetzt. Es ist teilweise vorteilhaft die Komponenten II und III im Molverhältnis 1:5, vorzugsweise 1:3 einzusetzen.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

Beispiel A

Myzus-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 3, 2, 4, 23, 11, 12, 9, 1, 14, 7, 8.

Beispiel B

Doralis-Test (systemische Wirkung)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 3, 2, 4, 23, 11, 12, 9, 1, 14, 8.

Beispiel C

Grenzkonzentrations-Test / Wurzelsystemische Wirkung

Testinsekt: Phaedon cochlearia-Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 7, 8.

Beispiel D

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 4, 7, 9.

Beispiel E

Grenzkonzentrations-Test / Bodeninsekten

| | | | |
|---|---|---|---|
| Testinsekt: | Phorbia antiqua-Maden (im Boden) | | |
| Lösungsmittel: | 3 | Gewichtsteile | Aceton |
| Emulgator: | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm ( = mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 9, 11, 12, 23.

Beispiel F

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 9.

Herstellungsbeispiele

Die Ausgangsverbindung II kann wie folgt hergestellt werden:

Zu einer Lösung von 65,6 g (0,4 Mol) 2,2-Dimethyl-2,3-dihydrobenzofuranol-(7) und 62,4 g (0,4 Mol) N-Bis-chlorcarbonyl-methylamin in 1,2 l Toluol tropft man unter Rühren 40,4 g (0,4 Mol) Triethylamin. Man rührt 8 Std. bei Raumtemperatur, saugt das ausgefallene Amin-hydrochlorid ab und dampft i. Vak. ein. Der Rückstand kristallisiert nach kurzer Zeit. Er wird mit Petrolether verrührt und abgesaugt. 113,4 g farblose Kristalle, Fp. 63°C.

Beispiel 1 (Verbindung Nr. 1)

2,3-Dihydro-2,2-dimethyl-7-benzofuranyl-N/2(N', N'-dimethylamino)ethoxycarbonyl7-N-methyl-carbamat.

7 g (0,025 Mol) Chlorcarbonylverbindung II und 2,2 g (0,025 Mol) Dimethylaminoethanol werden mit 2,1 g Natriumhydrogencarbonat 2 Tage in 60 ml Toluol gerührt. Die filtrierte Lösung wird mit Wasser gewaschen, die organische Phase über Natriumsulfat getrocknet und i.Vak. eingeengt. Letzte Lösungsmittelreste werden im Hochvakuum bei 40°C entfernt. 7,8 g farbloses Öl (92 % der Theorie) $n_D^{20}$=1.5121.

Beispiel 2 (Verbindung Nr. 2)

2,3-Dihydro-2,2-dimethyl-7-benzofuranyl-N-ethoxycarbonyl-N-methyl-carbamat.

7 g (0,025 Mol) Chlorcarbonylverbindung II und 1,2 g Ethanol in 100 ml Toluol oder 100 ml Methylenchlorid werden bei

Raumtemperatur mit 4 ml Triethylamin tropfenweise versetzt. Nach Beendigung der Zugabe rührt man noch 1 Stunde. Aufarbeitung wie Beispiel 1.
5,4 g farbloses Öl (73 % der Theorie) $n_D^{20}$=1.5165.

Beispiel 3 (Verbindung Nr. 7)

2,3-Dihydro-2,2-dimethyl-7-benzolfuranyl-N(2-ethylsulfinylethoxycarbonyl-N-methyl-carbamat

10,6 g (0,03 Mol) 2,3-Dihydro-2,2-dimethyl-7-benzofuranyl-N-(2-ethylthioethoxycarbonyl)-N-methyl-carbamate werden in 40 ml Eisessig bei 0°C tropfenweise mit 2,7 ml 40 %igem Wasserstoffsuperoxid versetzt. Am nächsten Tag gießt man die Mischung in 300 ml Wasser und extrahiert mit Methylenchlorid. Mit Natriumhydrogencarbonat wird die Lösung neutralisiert. Nach dem Filtrieren wird die organische Phase wie im Beispiel 1 beschrieben aufgearbeitet. 11 g farbloses Öl (98 % der Theorie) $n_D^{20}$=1.5320.

Beispiel 4 (Verbindung Nr. 23)

2,3-Dihydro-2,2-dimethyl-7-benzofuranyl-N-piperidinocarbonyl-N-methyl-carbamat

5,7 g (0,02 Mol) Chlorcarbonylverbindung II werden in 50 ml Methylenchlorid gelöst und mit 4 ml Piperidin (0,04 Mol) tropfenweise versetzt und dann 1 Stunde weitergerührt. Nach versetzen mit 100 ml Wasser wird die organische Phase abgetrennt und über Natriumsulfat getrocknet. Nach Einengen der Lösung kristallisiert das Produkt durch Zusatz von Petrolether. 5 g farblose Kristalle (73 % d. Th.)
Schmp. 98-100°C

Zusammenstellung der neuen Insektizide

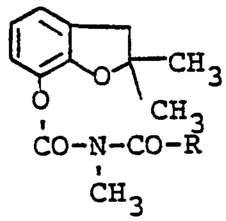

| Verbindung Nr. | R | $n_D^{20}$ | Schmp. °C |
|---|---|---|---|
| 1 | 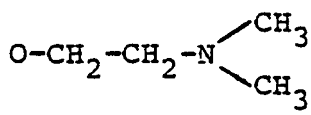 | 1.5120 | |
| 2 | $OCH_2-CH_3$ | 1.5165 | |
| 3 | $OCH_3$ | 1.5242 | |
| 4 | $O-CH_2-C\equiv CH$ | | 90 |
| 5 | $O-CH_2-CH_2-S-CH_2-CH_3$ | 1.5293 | |
| 6 | $O-CH_2-CH_2-O-CH_2-CH_3$ | 1.5133 | |
| 7 | $O-CH_2-CH_2-SO-CH_2-CH_3$ | 1.5320 | |
| 8 | $O-CH_2-CH_2-SO_2-CH_2-CH_3$ | 1.5260 | |
| 9 | $O-CH_2-CH_2-Cl$ | 1.5282 | |
| 10 | $O-CH_2-CF_3$ | 1.4871 | |
| 11 | $O-CH_2CCl_3$ | 1.5272 | |
| 12 | 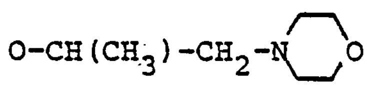 | 1.5218 | |
| 13 | 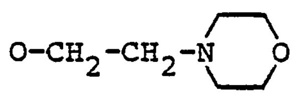 | 1.5180 | |
| 14 | 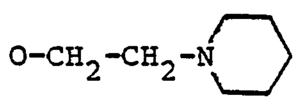 | 1.5258 | |
| 15 | $O-CH_2-CH_2-OH$ | 1.5283 | |

| Verbindung Nr. | R | $n_D^{20}$ | Schmp.°C |
|---|---|---|---|
| 16 | $NHCH_3$ | | 95-97 |
| 17 | $N(C_2H_5)_2$ | 1.5159 | |
| 18 | $N(iC_3H_7)_2$ | | 126 |
| 19 | $NH-CH_2-CH(CH_3)_2$ | | 80 |
| 20 | $NH-C_{18}-H_{37}$ | | 50 |
| 21 | $N(CH_2-CH=CH_2)_2$ | 1.5258 | |
| 22 | 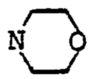 | | 113 |
| 23 | 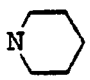 | | 98 - 100 |
| 24 | $-NH_2$ | | 136°-138 |

Patentansprüche

1. N-carboxylierte N-Methylcarbamidsäurearylester der Formel I

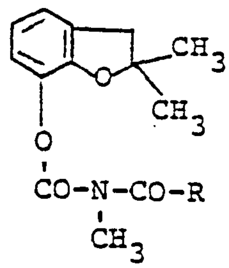

(I)

in welcher

R für einen geradkettigen oder verzweigten Alkoxyrest mit 1-10 C-Atomen, einen Alkenoxy- oder Alkinoxyrest mit 3-6 C-Atomen oder einen Cycloalkoxyrest mit 5-7 C-Atomen, die gegebenenfalls durch Halogen oder einen Rest der allgemeinen Formel $-X - R^1$ oder $-Y\ R^2R^3$ substituiert sein können; ferner steht R für $- Y\ R^2R^3$.

In der Formel $-X-R^1$ steht X für das Sauerstoff- oder Schwefelatom, sowie für die Sulfoxid- oder Sulfongruppe und $R^1$ für einen geradkettigen oder verzweigten Alkylrest mit 1-4 C-Atomen.

In der Formel $-Y\ R^2R^3$ steht Y für Stickstoff. $R^2$ und $R^3$ stehen für Wasserstoff und/oder verzweigte oder geradkettige Alkylreste mit 1 - 18 C-Atomen, Alkenylreste mit 3-8 C-Atomen, Alkinylreste mit

3-8 C-Atomen, Alkinylreste mit 3-8 C-Atomen. Außerdem kann die Formel $-Y\ R^2R^3$ für einen cyclischen Aminorest stehen, der durch die allgemeinen Formeln

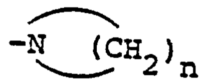 oder 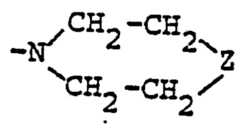

charakterisiert ist.

Hierbei steht n für 2-6 und Z für das Sauerstoff- oder Schwefelatom, sowie für ein wasserstoff- oder alkylsubstituiertes Stickstoffatom, wobei der Alkylsubstituent am Stickstoffatom 1-4 C-Atome haben kann.

2. Verfahren zur Herstellung der N-carboxylierte N-Methylcarbamidsäurearylester der Formel I, gemäß Anspruch 1, dadurch gekennzeichnet, daß man N-Chlorcarbonyl-N-methyl-2,2-dimethyl-2,3-dihydrobenzo-furanyl-(7)-carbamat der Formel II

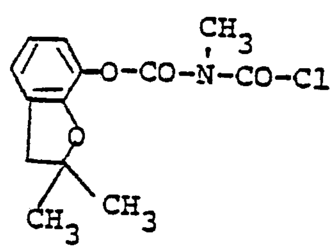

(II)

mit Alkoholen der Aminen der Formel III,

R - H    III

worin R die oben angegebene Bedeutung hat,

in Gegenwart eines säurebindenden Mittels sowie gegebenenfalls eines Verdünnungsmittels umsetzt.

3. Schädlingsbekämpfungsmittel gekennzeichnet durch einen Gehalt an mindestens einem N-carboxylierte N-Methylcarbamidsäurearylester der Formel (I).

4. Verwendung von N-carboxylierte N-Methylcarbamidsäurearylester der Formel (I) zur Bekämpfung von Schädlingen.

5. Verfahren zur Bekämpfung von Schädlingen, dadurch gekennzeichnet, daß man N-carboxylierte N-Methylcarbamidsäurearylester der Formel (I) auf die Schädlinge und/oder ihren Lebensraum einwirken läßt.

6. Verfahren zur Herstellung von Schädlingsbekämpfungsmitteln, dadurch gekennzeichnet, daß man N-Methylcarbamidsäurearylester der Formel (I) mit Streckmitteln und/oder oberflächenaktiven Mitteln vermischt.

Europäisches Patentamt

# EUROPÄISCHER RECHERCHENBERICHT

0022498

Nummer der Anmeldung
EP 80 10 3638

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int.Cl. 3) |
|---|---|---|---|
| D,X | DE - A - 2 132 936 (FARBENFABRIKEN BAYER A.G.)<br>* Insgesamt *<br>---- | 1-6 | C 07 D 307/86<br>A 01 N 47/22<br>47/34<br>47/38 |

RECHERCHIERTE SACHGEBIETE (Int. Cl. 3)

C 07 D 307/86

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-10-1980 | ALLARD |